# EUROPEAN PATENT SPECIFICATION

(11) **EP 3 948 294 B1**
(45) Date of publication and mention of the grant of the patent: **07.06.2023**
(21) Application number: 20715859.3
(22) Date of filing: 30.03.2020
(51) Int. Cl.: G01N 33/66, G01N 33/68, G01N 33/72

(54) **USE OF FOLLISTATIN IN TYPE 2 DIABETES RISK PREDICTION**
VERWENDUNG VON FOLLISTATIN ZUR RISIKOVORHERSAGE VON TYP-2 DIABETES
UTILISATION DE LA FOLLISTATINE DANS LA PRÉDICTION DU RISQUE DE DIABÈTE DE TYPE 2

(30) Priority: 28.03.2019 SE 1950381
(43) Date of publication of application: 09.02.2022
(73) Proprietor: Lundoch Diagnostics AB, 211 49 Malmö (SE)
(72) Inventor: DE MARINIS, Yang, 21149 Malmö (SE)
(74) Representative: Zacco Sweden AB
(86) International application number: PCT/EP2020/058971
(87) International publication number: WO 2020/193808

(56) References cited:
- WO-A1-2006/066263
- WO-A2-2010/057135
- J. HANSEN ET AL: "Plasma follistatin is elevated in patients with type 2 diabetes: relationship to hyperglycemia, hyperinsulinemia, and systemic low-grade inflammation : Plasma Follistatin and Type 2 Diabetes", DIABETES/METABOLISM RESEARCH AND REVIEWS, vol. 29, no. 6, 1 September 2013 (2013-09-01), pages 463-472, XP055704173, GB ISSN: 1520-7552, DOI: 10.1002/dmrr.2415
- CHUNXIA ZHAO ET AL: "Overcoming Insulin Insufficiency by Forced Follistatin Expression in [beta] -cells of db/db Mice", MOLECULAR THERAPY : THE JOURNAL OF THE AMERICAN SOCIETY OF GENE THERAPY, vol. 23, no. 5, 1 May 2015 (2015-05-01), pages 866-874, XP055525294, US ISSN: 1525-0016, DOI: 10.1038/mt.2015.29
- JAKOB S. HANSEN ET AL: "Circulating Follistatin Is Liver-Derived and Regulated by the Glucagon-to-Insulin Ratio", JOURNAL OF CLINICAL ENDOCRINOLOGY AND METABOLISM, vol. 101, no. 2, 1 February 2016 (2016-02-01), pages 550-560, XP055704179, US ISSN: 0021-972X, DOI: 10.1210/jc.2015-3668

## Description

### TECHNICAL FIELD

Herein is presented novel means for type 2 diabetes risk assessment, with good predictive value up to four years before disease onset, using follistatin as a marker in a model incorporating at least one of three known blood biomarkers for type 2 diabetes, including HbA_{1c}, proinsulin, and C-peptide.

### BACKGROUND

The absolute global economic burden of diabetes is estimated to increase from U.S. $1.3 trillion in 2015 to $2.5 trillion (2.4-2.6) by 2030, which counts for 2.2% of global GDP^{[1]}. In the US alone, the average medical expenditures of a patient with diagnosed diabetes is $16,752, which is about 2.3 times higher than what expenditures would be in the absence of diabetes. In some health care systems, diabetes patient care accounts for 25% of the entire costs.

It is possible to prevent type 2 diabetes (T2D) through lifestyle intervention if disease risk could be detected early enough^{[2]}. Currently, oral glucose tolerance test (OGTT) and fasting plasma glucose (FPG) have been used to assess diabetes risks. However, diabetes and even complications may have already occurred by the time abnormal glucose levels are detected. Furthermore, diabetes is a systemic disease and may result in changes of multiple blood signatures, which makes it questionable to assess diabetes risk based on only glucose. Nevertheless, in current clinical practice, a potential diabetes diagnosis is solely assessed by glucose measurements: a patient is with either high blood glucose levels (diabetic) or normal glucose levels (non-diabetic). However, among the "non-diabetics", each individual may have different risk levels of developing diabetes in the future, which presently cannot be assessed efficiently by available techniques and biological marker measurements. It has therefore been found appropriate to assess type 2 diabetes risk by multivariable individualized risk scores.

In the present report and study, the inventor reports the development of means for clustering individuals without diabetes into different risk groups incorporating different biomarkers. Furthermore, the inventor has established a mathematical model that could accurately predict diabetes risks.

Surprisingly it is found, that follistatin can be used as a biomarker for early diagnosis of type 2 diabetes, which use is herein reported. Further, a method of composing a biomarker signature for the early prediction of type 2 diabetes in a human is herein disclosed.

### BRIEF DESCRIPTION OF THE DRAWINGS

- Fig. 1:: Liver cell follistatin secretion is controlled by GCKR-GCK complex.
- Fig. 2:: Diabetes progression cohort clustering.
- Fig. 3:: The importance of the five variables (plasma follistatin, proinsulin, insulin, C-peptide, baseline HbA_{1c}) and selection of the variables (Fig 3A, 3B, 3C).
- Fig. 4:: Performance and validation of four models to assess risk of 4-year incidence of type 2 diabetes in the cohort.
- Fig. 5:: The ROC curve of the selected model (NNET) with four biomarkers of 10-fold cross-validation.
- Table 1:: ROC AUC of 10-fold cross validation with different variables by different methods.
- Table 2:: Performance of each cluster with 10-fold cross validation.
- Table 3:: Comparison of AUCs between models with and without follistatin for each class.

### DETAILED DESCRIPTION

The present invention is defined in the appended claims and builds on the surprising realization by the inventor that follistatin is a biomarker for short-term, high-risk, development of type 2 diabetes in a human.

The present invention accordingly relates to the diagnosis and/or the prediction for an individual human of having a high risk of developing type 2 diabetes within a short time period of in less than 10 years, such as in less than 9 years, less than 8 years, less than 7 years, less than 6 years, less than 5 years, or less than 4 years, if his or hers condition is left untreated. A particular advantage of early diagnosis and/or prediction is the ability to prevent disease occurrence by preventive treatment.

Hence in a first embodiment and aspect of the invention there is detailed the use of follistatin as a biomarker in a method of diagnosing short-term, high-risk, development of type 2 diabetes in a human.

In another aspect of the first embodiment and aspect of the invention, there is detailed the use of follistatin a biomarker in a method of predicting short-term, high-risk, development of type 2 diabetes in a human.

In an embodiment of the first aspect, there is detailed the use of follistatin according to the first embodiment, wherein the method of diagnosing and/or predicting short-term, high-risk, development of type 2 diabetes in a human is a high-risk development of type 2 diabetes in less than 10 years, preferably in less than 9 years, less than 8 years, less than 7 years, less than 6 years, less than 5 years, or less than 4 years.

In an embodiment of the first aspect, there is detailed the use of follistatin according to any previous embodiment, wherein the method of diagnosing and/or predicting short-term, high-risk, development of type 2 diabetes in a human comprises k-means clustering to assess type 2 diabetes progression risk levels using at least one further biomarker selected from baseline HbA_{1c}, proinsulin, or C-peptide, preferably, at least two or at least 3 further biomarkers selected from of baseline HbA_{1c}, proinsulin, or C-peptide.

In an embodiment of the first aspect, there is detailed the use of follistatin according to any previous embodiment, wherein the method of diagnosing and/or predicting short-term, high-risk, development of type 2 diabetes in a human comprises evaluating available biomarkers by recursive feature elimination for building a risk prediction model.

In an embodiment of the first aspect, there is detailed the use of follistatin according to any previous embodiment, wherein the method of diagnosing and/or predicting short-term, high-risk, development of type 2 diabetes in a human comprises measuring blood levels from said human of follistatin and at least one further biomarker selected from baseline HbA_{1c}, proinsulin, or C-peptide, and comparing the measured blood levels with a model value based on averaged blood level values from a group of humans having a high risk of developing type 2 diabetes in less than 10 years, preferably in less than 5 year, or more preferably, in less than 4 years.

In an embodiment of the first aspect, there is detailed the use of follistatin according to any previous embodiment, wherein the method of diagnosing and/or predicting short-term, high-risk, development of type 2 diabetes in a human comprises measuring blood levels of at least two, or at least 3 further biomarkers selected from of baseline HbA_{1c}, proinsulin, or C-peptide.

In an embodiment of the first aspect, there is detailed the use of follistatin according to any previous embodiment, wherein the method of diagnosing and/or predicting short-term, high-risk, development of type 2 diabetes in a human is a method of predicting short-term, high-risk, development of type 2 diabetes in a human.

In a second aspect of the invention there is detailed a method of composing a biomarker signature for the early prediction of type 2 diabetes in a human, comprising measuring blood levels from said human of follistatin and at least one further biomarker selected from baseline HbA_{1c}, proinsulin, or C-peptide, and comparing the measured blood levels with a model value based on averaged blood level values from a group of humans having a high risk of developing type 2 diabetes in less than 10 years, preferably in less than 5 year, or more preferably, in less than 4 years.

In an embodiment of the second aspect, blood levels of at least two, or at least 3 further biomarkers selected from of baseline HbA_{1c},proinsulin, or C-peptide are measured.

Likewise, there is herein detailed follistatin for use as a biomarker in the diagnosis and/or predicting of short-term, high-risk, development of type 2 diabetes in a human.

In an embodiment of thereof, there is detailed follistatin for use as a biomarker in the diagnosis and/or predicting of short-term, high-risk, development of type 2 diabetes in a human, wherein short-term, high-risk, development of type 2 diabetes in a human is a high-risk development of type 2 diabetes in less than 10 years, preferably in less than 9 years, less than 8 years, less than 7 years, less than 6 years, less than 5 years, or less than 4 years.

In an embodiment of thereof, there is detailed follistatin for use as a biomarker in the diagnosis and/or predicting of short-term, high-risk, development of type 2 diabetes in a human, wherein diagnosis of short-term, high-risk, development of type 2 diabetes in a human comprises k-means clustering to assess type 2 diabetes progression risk levels using at least one further biomarker selected from baseline HbA_{1c}, proinsulin, or C-peptide, preferably, at least two, or at least 3 further biomarkers selected from of baseline HbA_{1c}, proinsulin, or C-peptide.

In an embodiment of thereof, there is detailed follistatin for use as a biomarker in the diagnosis and/or predicting of short-term, high-risk, development of type 2 diabetes in a human, wherein diagnosis of short-term, high-risk, development of type 2 diabetes in a human comprises evaluating available biomarkers by recursive feature elimination in a risk prediction model.

In an embodiment of thereof, there is detailed follistatin for use as a biomarker in the diagnosis and/or predicting of short-term, high-risk, development of type 2 diabetes in a human comprising composing a biomarker signature for the early prediction of type 2 diabetes in a human, comprising measuring blood levels of follistatin and at least one further biomarker selected from baseline HbA_{1c}, proinsulin, or C-peptide, and comparing the measured blood levels with a model value based on averaged blood level values from a group of humans having a high risk of developing type 2 diabetes in less than 10 years, preferably in less than 5 years, or more preferably, in less than 4 years.

In an embodiment of thereof, there is detailed follistatin for use as a biomarker in the diagnosis and/or predicting of short-term, high-risk, development of type 2 diabetes in a human, further comprising measuring blood levels of at least two, or at least 3 further biomarkers selected from of baseline HbA_{1c}, proinsulin, or C-peptide.

The present invention relates to the diagnosis and identification of an individual having a high risk of developing type 2 diabetes within a short time period of in less than 10 years, such as in less than 9 years, less than 8 years, less than 7 years, less than 6 years, less than 5 years, or less than 4 years, if his or hers condition is left untreated. A particular advantage of early diagnosis is the ability to prevent disease occurrence by preventive treatment.

In accordance with the invention, the present invention in embodiments relates to diagnosing and/or predicting the risk of developing type 2 diabetes in a human in less than 10 years, such as in less than 9 years, less than 8 years, less than 7 years, less than 6 years, less than 5 years, or less than 4 years, if his or hers condition is left untreated.

In an embodiment, a high risk of developing type 2 diabetes within a short time period is present, when an individual presents with follistatin of at least 2000 pg/mL in blood serum.

In an embodiment thereof, a high risk of developing type 2 diabetes within a short time period is present, when an individual further presents with proinsulin of at least 20 pmol/L in blood serum.

In an embodiment thereof, a high risk of developing type 2 diabetes within a short time period is present, when an individual further presents with C-peptide of at least 5 ng/mL in blood serum.

In an embodiment thereof, a high risk of developing type 2 diabetes within a short time period is present, when an individual further presents with insulin of at least 800 pg/mL in blood serum.

As documented in the below examples, individuals presenting fasting with blood serum levels of follistatin or follistatin and one or more of the above biological markers as given above and in the examples, were observed to develop type 2 diabetes as measured using HbA_{1c} and 48-months HbA_{1c} with statistical significance over a control population comprising non-progressing pre-diabetic and non-diabetic individuals, and are therefore a population having a high risk of developing type 2 diabetes. By themselves, with the notable exemption of follistatin, each other marker was not statistically distinguishable between the populations, but the combination of markers provided clear identification. For follistatin, blood serum levels above 2500 pg/mL, preferably above 3000 pg/mL, by itself was significant in indicating a high risk of developing type 2 diabetes in the individual examined.

### EXAMPLES

### Objective of the reported study

The purpose of this study was to develop a prediction model to assess type 2 diabetes (T2D) risk by blood biomarker signature.

### Research design and methods

Study individuals are from a longitudinal cohort, which includes 152 non-diabetes participants with four-year follow-up for T2D progression. The cohort was clustered by k-means to assess T2D progression risk levels using baseline HbA_{1c}, proinsulin, C-peptide, follistatin and 48-month HbA_{1c}. Available biomarkers were evaluated by recursive feature elimination to build the risk prediction model. T2D four-year prediction based on the risk clustering was tested by Neural Network (NNET), Support Vector Machine (SVM), Random Forest (RF) and Generalize Logistic Regression (GLM) machine learning methods. The performance of the four candidate risk models were evaluated using 10-fold cross validation.

### General results

The cohort was clustered into three risk groups: high risk, intermediate risk and low risk. Baseline HbA_{1c}, proinsulin, C-peptide and follistatin were selected after biomarker validation. An optimal model for assessing an individual's 4-year risk of developing T2D was developed by NNET machine learning method using these four biomarkers. The areas under curve (AUCs) of the receiver-operating characteristic (ROC) curve for prediction of each risk group are 0.9 (high risk), 0.96 (intermediate risk) and 0.99 (low risk), respectively (10-fold cross-validation). The mean AUC of the three risk groups is 0.97.

Accordingly, herein is presented novel means for type 2 diabetes risk assessment four years before disease onset with a model incorporating four blood biomarkers including HbA_{1c}, proinsulin, C-peptide and follistatin.

### METHODS

### Cohort participants

The cohort was a multicenter, randomized, double-blind, placebo control of a clinical trial recruited in the US. HbA_{1c} was measured and co-medications were documented in patients at baseline, 1 year, 2 year, and 4 year. A T2D-progression sub-cohort of approximately 400 patients was selected from the cohort based on patient HbA_{1c} change from baseline over the 4-year trial period and absence of diabetes drug administration to these patients.

### PLASMA PROTEIN BIOMARKER MEASUREMENTS

Fasting insulin, Pro-insulin, C-peptide, and Follistatin were measured by enzyme-linked immunosorbent assay (ELISA) in baseline EDTA-plasma samples obtained from 314 patients selected to be included in the type 2 diabetes progression cohort. In all assays except C-peptide, samples were measured in technical replicates and subsequent intra-assay average coefficient of variation (%CV) was calculated. Inter-assay %CV was calculated using internal controls. Insulin concentrations were determined using a custom-built electro-chemiluminescence immunoassay and a MESO QuickPlex SQ 120 (Meso Scale Discovery (MSD), Gaithersburg, MD) following an internally optimized protocol^{[3-5]}. The intra-assay CV was 10.9%, and the inter-assay CV was 3.1% for insulin assays. C-peptide levels were quantified with an electro-chemiluminescence immunoassay using the Cobas e411 (Roche Diagnostics, Mannheim, Germany). Intact Pro-insulin was measured after a 4-fold dilution in sample buffer using a colorimetric ELISA with calibrators against WHO 1st International Standard for Pro-insulin following the manufacturer's instructions (IRP 84/611; catalog # IV2-102E, Immuno-Biological Laboratories, Inc., Minneapolis, MN). Assay absorbance was measured using a PHERAstar FSX (BMG Labtech Inc., Cary, NC). Intra-assay CV was 6.6% and inter-assay CV was 10% for Pro-insulin assays. Plasma Follistatin levels were measured after 2-fold dilution in sample diluent using a colorimetric ELISA according to the manufacturer's instructions (catalog # DFN00, R&D Systems, Minneapolis, MN) . Assay absorbance was measured using a PHERAstar FSX (BMG Labtech Inc., Cary, NC). The intra-assay CV was 2.1%, and the inter-assay CV was 10% for Follistatin assays.

### Model development process: Using machine learning to devise the 4-year type 2 diabetes-risk

All statistical analyses were performed using the statistical analysis software package the machine learning toolkit (Caret package^{[6]}) and the statistical computing environment R^{[7]}. Significance for the results was set at p≤0.05.

Feature selection was performed by recursive feature elimination method implemented in the machine learning R package Caret (e.g. rfe, rfefilter) to identify those blood parameters with the best prediction performance. The five candidate biomarkers were evaluated for inclusion in multi-marker models. Because predictive techniques can perform differently on data, we choose to use four different types of predictive models using the Caret package^{[6]} within the R environment^{[7]}. Four machine approaches were incorporated including NNET (Neural network), SVM (Support vector machines), RF (Random forest methods), and GLM (Generalized logistic regression). Multivariate data analysis (NNET, SVM, RF, and GLM) was used to investigate whether a blood-based biomarker panel allows prediction of risk of developing type 2 diabetes. The best-performing NNET model was evaluated in a 10-fold cross-validation procedure to ensure the robustness of the results. The following characteristic numbers were calculated: AUC, accuracy, sensitivity and specificity.

Finally, receiver-operating characteristic (ROC) curves were created. DeLong's test using roc test of R library pROC^{[8]} for two correlated ROC curves (i.e. the ROC curves of high risk by NNET and NNET without Follistatin) was performed. P values <0.05 were considered to be significant.

The results showed that the outcome of the short-term, high-risk prediction was model independent, hence reflecting actual biological processes underlying the statistics of the studied groups.

### RESULTS

To identify genetic factors that influence plasma follistatin levels, we performed GWAS on two further, different cohorts. Glucokinase regulatory protein (GCKR) was identified as the genetic regulator of plasma follistatin levels. Here we show that GCKR regulates liver follistatin secretion together with glucagon and insulin in a human hepatocyte cell line HepG2. Previous investigations have shown that GCKR forms a tight complex with GCK in the nucleus, and dissociation of the GCK-GCKR binding leads to increased GCK translocation from nucleus to cytoplasm, which regulates liver cell glucose uptake and glycolysis.

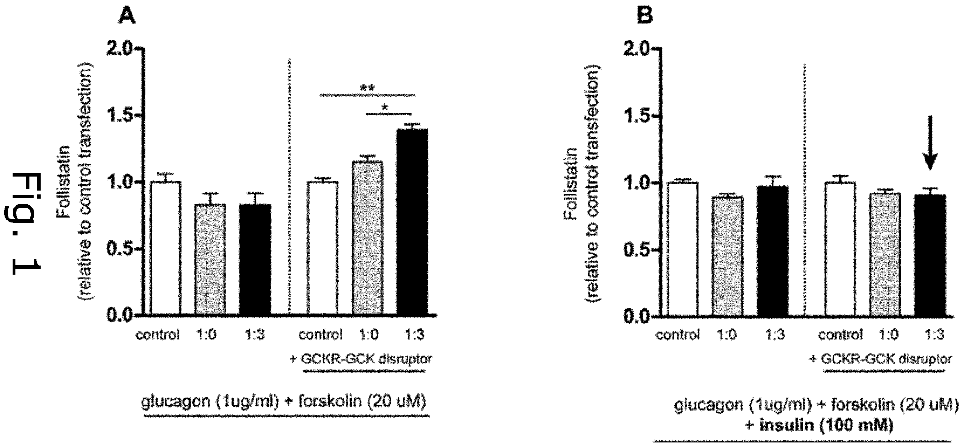

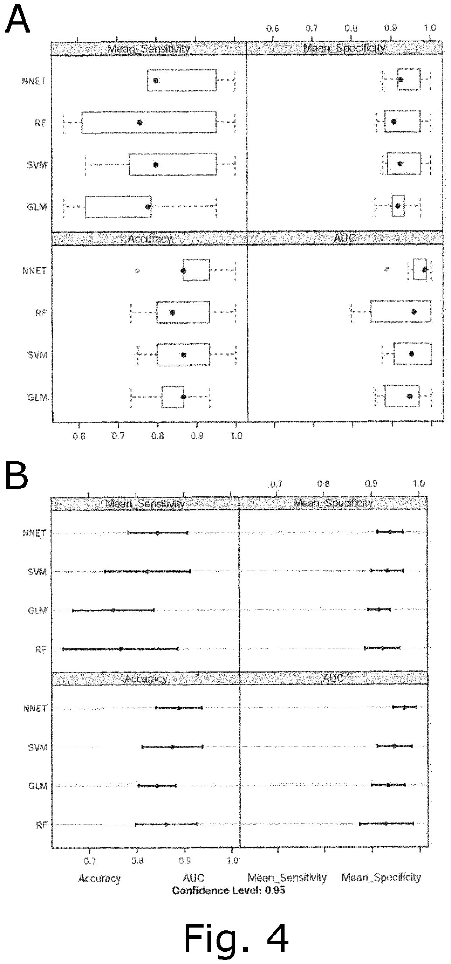

In this study, HepG2 cells were transfected with GCK, or co-transfected with GCK and GCKR expressing plasmids (1:3 molar ratio). In addition, cells were treated with and without AMG-3669, a GCK-GCKR complex disruptor molecule that promotes strong translocation of disassociated GCK from the nucleus to cytoplasm. Cells were incubated with glucagon (1 µg/ml) and intracellular cAMP activator forskolin (20 µM) in low glucose DMEM medium (5.5 mM), conditions previously shown to stimulate follistatin secretion in liver cells^{[11]}. In the presence of the GCK-GCKR complex and its disruptor AMG-3969, follistatin secretion increased by 40% compared to control (Fig. 4A), which was reversed by co-incubation with insulin (Fig. 4B). Transfection with GCK alone, or GCK-GCKR co-transfection without AMG-3969, which does not affect translocation of GCKR from nucleus to cytoplasm, had no effect on follistatin secretion (Fig. 1).

**Figure 1****. Liver cell follistatin secretion is controlled by GCKR-GCK complex**. A. Human liver carcinoma-derived HepG2 cells were transfected with the indicated plasmids: i) control (pCMV-XL4, open bars); ii) GCK:GCKR (1:0; no GCKR, grey bars); iii) GCK:GCKR (1:3, black bars). Forty-eight hours after transfection, the cells were serum starved in low glucose (5.5 mM) DMEM for 3 hrs, and a GCKR-GCK disruptor molecule AMG-3969 (0.7 µM) was added in the media for 30 min. Cells were then incubated in serum-free low glucose (5.5 mM) DMEM containing glucagon (1 µg/ml) and forskolin (20 µM), and AMG-3969 (0.7 µM) was added to the corresponding wells. After 4 hrs incubation, the media was collected for follistatin assay by ELISA. The follistatin levels were normalized to the protein concentration within each sample. Two independent experiments with 3 technical replicates per condition were performed in different days using different plasmid preparations and cell passage numbers. B. HepG2 cells were treated as described in panel A, but in the presence of insulin (100 nM). * p<0.05 and **p<0.01 as indicated.

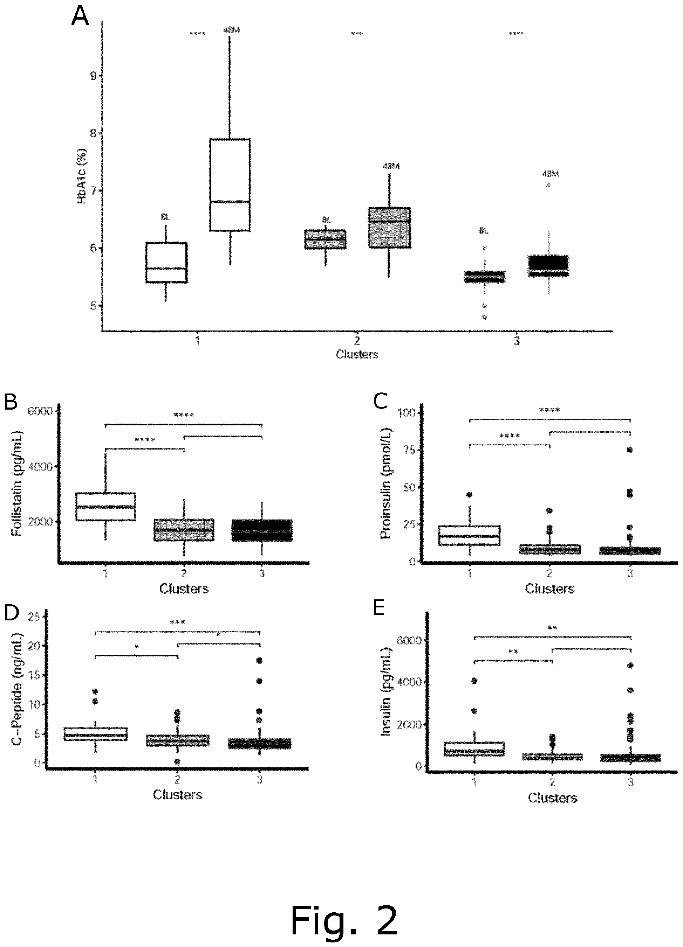

To better characterize type 2 diabetes risks among individuals without diabetes, the US cohort participants were clustered using follistatin and other variables that have been previously shown to be associated with diabetes or future diabetes risks. K-means clustering using baseline HbA_{1c}, proinsulin, C-peptide, follistatin and 48-month HbA_{1c} identified three risk groups: high risk, intermediate and low risk groups (Fig. 2). High risk group in cluster 1 progressed to diabetes from non-diabetes after 48 months, with increased median HbA_{1c} from 5.6% at baseline to 6.8% at 48 months; intermediate risk group in cluster 2 represents non-progressing pre-diabetes (median HbA_{1c} baseline 6.2% to HbA_{1c} 48-month 6.3%) and low risk group in cluster 3 included non-progressing non-diabetic individuals (median HbA_{1c} baseline 5.4% to HbA_{1c} 48-month 5.5%) (Fig. 2A). Patients from cluster 1 had significantly higher plasma follistatin levels at baseline than other clusters, 48 months before diabetes onset (Fig. 2B), as well as higher baseline plasma proinsulin (Fig. 2C), C-Peptide (Fig. 2D) and insulin levels (Fig. 2E).

**Figure 2****. Diabetes progression cohort clustering.** Individuals from the US cohort (n=152) were clustered by unsupervised K-means using baseline HbA_{1c}, plasma Follistatin, Pro-insulin, C-peptide and HbA_{1c} at 48 months. A. Cluster1: progression from non-diabetes to diabetes (open bars; median HbA_{1c} baseline 5.6% to HbA_{1c} 48-month 6.8%; n=20); cluster2: pre-diabetes non-progressing (grey bars; median HbA_{1c} baseline 6.2% to HbA_{1c} 48-month 6.3%; n=62); clusters: non-diabetic non-progressing (black bars; median HbA1c baseline 5.4% to HbA_{1c} 48-month 5.5%; n=70). B-E. Cluster distribution of baseline Follistatin (pg/mL, B), Pro-insulin (pmol/L, C), C-peptide (ng/mL, D) and Insulin (pg/mL, E). BL: Baseline; 48M: HbA_{1c} 48-month. ****p<0.0001, *** p<0.001, **p<0.01, *p<0.05 as indicated.

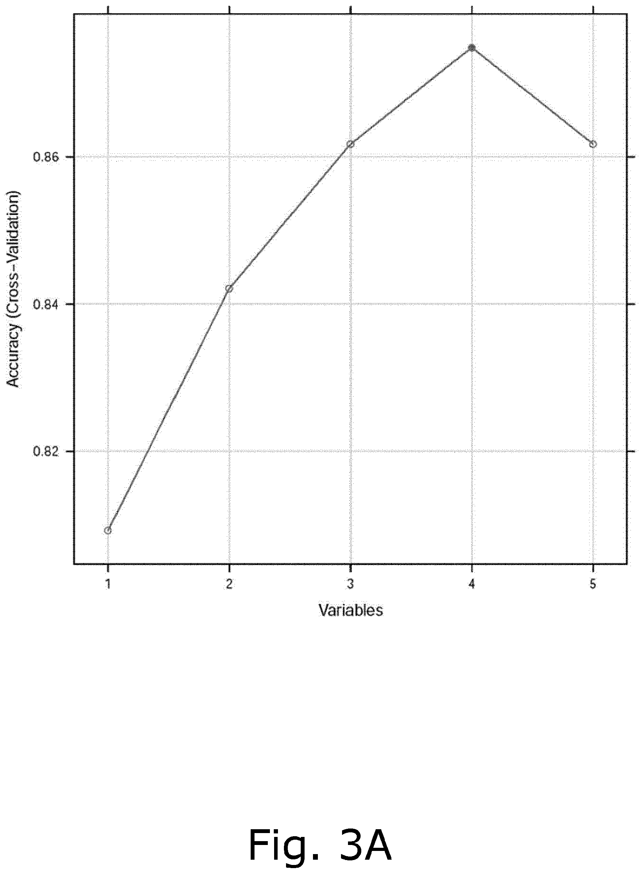

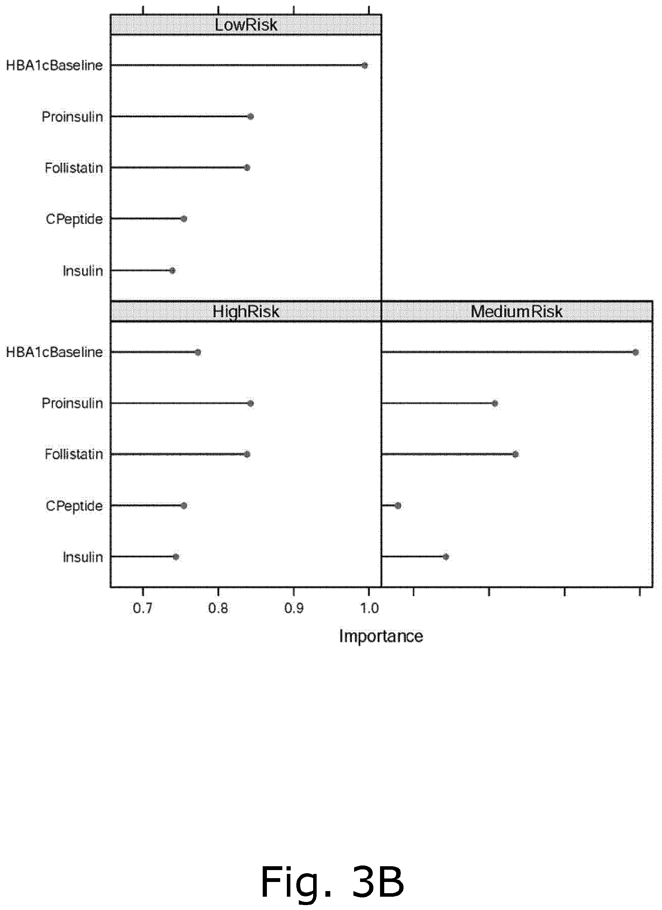

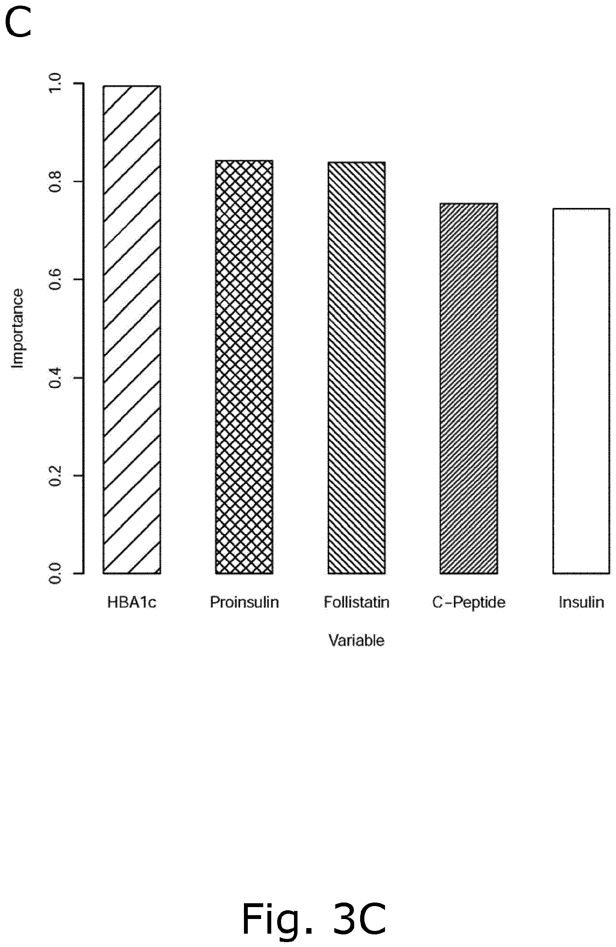

To validate the prediction power of each baseline variable (HbA_{1c}, proinsulin, C-peptide, insulin and follistatin) and their combination, we performed recursive feature elimination using the rfe-function of Caret^{[6]} by Neural Network (NNET), Support Vector Machine (SVM), Random Forest (RF) and Generalize Logistic Regression (GLM) machine learning methods (Table 1). For four-year type 2 diabetes prediction of each risk groups, proinsulin and follistatin have the highest importance for high-risk group, HbA_{1c} and follistatin for intermediate and low risk group (Fig. 3B). The overall importance for the three risk groups are presented by max operation (Fig. 3C). Combination of four variables gives the highest ref accuracy (10-fold cross-validation, Fig. 3A). Finally, four top risk factors (baseline HbA_{1c}, follistatin, proinsulin and C-peptide) were selected as the candidate biomarkers.

**Figure 3****. The importance of the five variables (plasma follistatin, proinsulin, insulin, C-peptide, baseline HbA_{1c}) and selection of the variables.** The accuracy with different variable using recursive feature elimination (3A), the contribution of each variable in different risk levels (3B), and the max score of the three risk levels for each variable (3C).

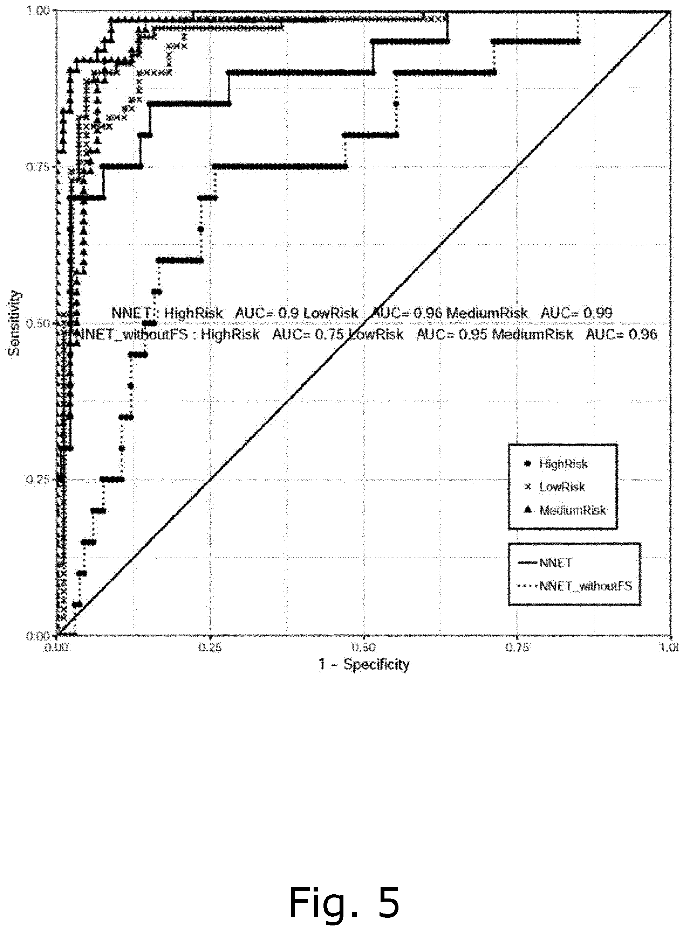

Different machine learning methods were then compared to study prediction performance of four-year type 2 diabetes risks incorporating the selected biomarkers. NNET, SVM, RF and GLM machine learning methods were evaluated by 10-fold cross-validation (Table 1). The ranges of sensitivity, specificity, accuracy, and AUC (Fig. 4A) and confidence intervals (Fig. 4B) were compared between the four models. NNET remained stable and performed substantially better than other three models in sensitivity and specificity. The mean value of Sensitivity and Specificity are greater than 0.84 (Table 2). Furthermore, in comparison of accuracy and AUC among the four models, NNET got a higher performance. The receiver-operating characteristic (ROC) curve and the area under curve (AUC) is 0.9 for high risk group, 0.96 for intermediate risk group and 0.99 for low risk group are 0.9, 0.96, and 0.99, respectively (10-fold cross validation). Adding follistatin to this NNET model improved the AUC of high-risk group significantly (AUC 0.9 <with follistatin> vs. 0.75 <without follistatin>, P = 4e-04 DeLong's test). For intermediate risk, the AUCs improved as (AUC 0.99 <with follistatin> vs. 0.96 <without follistatin>, P = 1e-02 DeLong's test), whereas for the low risk it is (AUC 0.96 <with follistatin> vs. 0.95 <without follistatin>, P = 1e-01 DeLong's test), respectively (Table 3 and Fig. 5).

**Figure 4****. Performance and validation of four models to assess risk of 4-year incidence of type 2 diabetes in the cohort.** The ranges of sensitivity, specificity, accuracy, and AUC (4A) and the confidence levels (4B) are shown for the four models.

**Figure 5****. The ROC curve of the selected model (NNET) with four biomarkers of 10-fold cross-validation.** ROC curves are presented for NNET model incorporating the four biomarkers (HbA_{1c} of baseline, follistatin, proinsulin, and C-peptide) on diabetes risk groups (high, medium and low risk) on the cohort dataset (10-fold cross validation). DeLong's test for the ROC curves of signature with and without follistatin is p=4e-04 for high risk group, p=0.01 for intermediate risk group, and p=0.1 for low risk group.

### Discussion

Base on the results in "risk clustering analysis", we used four variables to compose a biomarker signature to predict future diabetes: baseline follistatin, HbA_{1c}, pro-insulin and C-Peptide.

A multi-biomarker model was developed to assess risk of type 2 diabetes by four blood b biomarkers using multiple statistical approaches. The performance of the NNET model is better than that of any other baseline measure of risk. This NNET model provides a more convenient alternative for obtaining a risk estimate: a laboratory would measure the biomarker concentrations in a fasting blood sample and return the computed risk level. This NNET model does not depend on anthropometries or self-reported risk factors (such as family history or tobacco use).

The four biomarkers selected for the NNET model are involved in various biological pathways. Pro-insulin are critical indicators of metabolic disorders including diabetes and obesity. It has been shown that the disproportionate secretion of Pro-insulin, the precursor of insulin, can be not only a specific indicator of insulin resistance but also a hallmark of β-cell dysfunction^{[9]} . Follistatin is a secreted protein that is expressed in almost all major tissues, and studies have suggested that follistatin is linked to metabolic diseases^{[10,11]} with elevated plasma levels in patients with type 2 diabetes^{[10]}. Circulating follistatin has direct effects on glucose metabolism in humans by increasing insulin, and suppressing glucagon secretion from the pancreas^{[12]}. But it was previously unknown if follistatin predicts type 2 diabetes incidence prior to type 2 diabetes onset as demonstrated in the present disclosure.

Local overexpression of follistatin in the pancreas of diabetic mice resulted in increased serum insulin levels^{[13]}. A recent study by Tao et al. has identified follistatin as a mediator of systemic metabolic dysregulation associated with diabetes^{[14]}. In hyperglycemic mice and high-fat-fed obese mice, knockdown of follistatin restored glucose tolerance, white adipose tissue insulin signaling and suppression of hepatic glucose production by insulin. Previously, it was unknown that the secretion of follistatin from the liver is regulated by GCKR together with glucagon and insulin as demonstrated in this disclosure (Figure 1).

In obese individuals with diabetes who underwent gastric bypass surgery, serum follistatin decreased in parallel with HbA_{1c} levels. HbA_{1c} is measured primarily to identify the three-month average plasma-glucose concentration and thus can be used as a diagnostic test for diabetes.

It is found that a positive association between serum C-peptide levels and the risks of diabetes and pre-diabetes among Chinese women with a history of gestational diabetes.

The previous finding suggested that elevated C-peptide levels may be a predictor of diabetes and pre-diabetes^{[15]}.

The variables were run in several mathematic models using machine-learning methods: SVM, NNET, RF, and GLM. Among all tested methods, NNET gave the best performance. Using the selected biomarker signature, NNET predicts if the individual is at low, intermediate or high risk of developing diabetes in four years with very high specificity and sensitivity. The AUC is 0.9 (10-fold cross validation) to predict high risk, 0.99 to predict intermediate risk, and 0.96 to predict low risk (Fig. 3). The comparison of the AUC between the model with and without follistatin showed that the multiple biomarkers performed better than that with the single biomarker and without follistatin.

In summary, by applying a variety of statistical methods for biomarker selection, we developed a NNET model that incorporates up to four circulating biomarkers. This NNET provides superior assessment of diabetes risk compared with single biomarker alone and the model without Follistatin. The current results suggest this NNET model could be an important tool for identifying the individuals at highest risk of developing type 2 diabetes, a population for whom the most comprehensive prevention strategies should be considered. The improved performance of this model compared with that of single markers demonstrates the value of risk assessment models that incorporate multiple biomarkers including Follistatin from diverse pathophysiological pathways associated with type 2 diabetes.

### REFERENCES

[1] Bommer, C. et al. Global Economic Burden of Diabetes in Adults: Projections from 2015 to 2030. Diabetes Care 41, 963-970, doi:10.2337/dc17-1962 (2018).
[2] Knowler, W. C. et al. Reduction in the incidence of type 2 diabetes with lifestyle intervention or metformin. N Engl J Med 346, 393-403, doi:10.1056/NEJMoa012512 (2002).
[3] Lin, H. V. et al. GPR142 Controls Tryptophan-Induced Insulin and Incretin Hormone Secretion to Improve Glucose Metabolism. PLoS One 11, e0157298, doi:10.1371/ journal.pone.0157298 (2016).
[4] Bueno, A. B. et al. Positive Allosteric Modulation of the Glucagon-like Peptide-1 Receptor by Diverse Electrophiles. J Biol Chem 291, 10700-10715, doi:10.1074/jbc.M115.696039 (2016).
[5] Farb, T. B. et al. Regulation of Endogenous (Male) Rodent GLP-1 Secretion and Human Islet Insulin Secretion by Antagonism of Somatostatin Receptor 5. Endocrinology 158, 3859-3873, doi:10.1210/en.2017-00639 (2017).
[6] Kuhn, M. Caret: classification and regression training. Astrophysics Source Code Library (2015).
[7] Team, R. C. R: A Language and Environment for Statistical Computing. dim (ca533) 1, 34 (2018).
[8] Robin, X. et al. pROC: an open-source package for R and S+ to analyze and compare ROC curves. BMC bioinformatics 12, 77 (2011).
[9] Russo, G. T. et al. Factors associated with beta-cell dysfunction in type 2 diabetes: the BETADECLINE study. PLoS One 9, e109702 (2014).
[10] Hansen, J. et al. Plasma follistatin is elevated in patients with type 2 diabetes: relationship to hyperglycemia, hyperinsulinemia, and systemic low-grade inflammation. Diabetes Metab Res Rev 29, 463-472, doi:10.1002/dmrr.2415 (2013).
[11] Yndestad, A. et al. A complex role of activin A in non-alcoholic fatty liver disease. Am J Gastroenterol 104, 2196-2205, doi:10.1038/ajg.2009.318 (2009).
[12] Hansen, J. S. et al. Circulating Follistatin Is Liver-Derived and Regulated by the Glucagon-to-Insulin Ratio. J Clin Endocrinol Metab 101, 550-560, doi:10.1210/jc.2015-3668 (2016).
[13] Zhao, C. et al. Overcoming Insulin Insufficiency by Forced Follistatin Expression in beta-cells of db/db Mice. Mol. Ther. 23, 866-874, doi:10.1038/mt.2015.29 (2015).
[14] Tao, R. et al. Inactivating hepatic follistatin alleviates hyperglycemia. Nat Med 24, 1058-1069, doi:10.1038/s41591-018-0048-0 (2018).
[15] Yin, P. et al. C-peptide levels and the risk of diabetes and pre-diabetes among Chinese women with gestational diabetes. Journal of diabetes and its complications 31, 1658-1662 (2017).

### CLOSING COMMENTS

The term "comprising" as used in the claims does not exclude other elements or steps. The term "a" or "an" as used in the claims does not exclude a plurality. Although the present invention has been described in detail for purpose of illustration, it is understood that such detail is solely for that purpose, and variations can be made therein by those skilled in the art without departing from the scope of the invention.

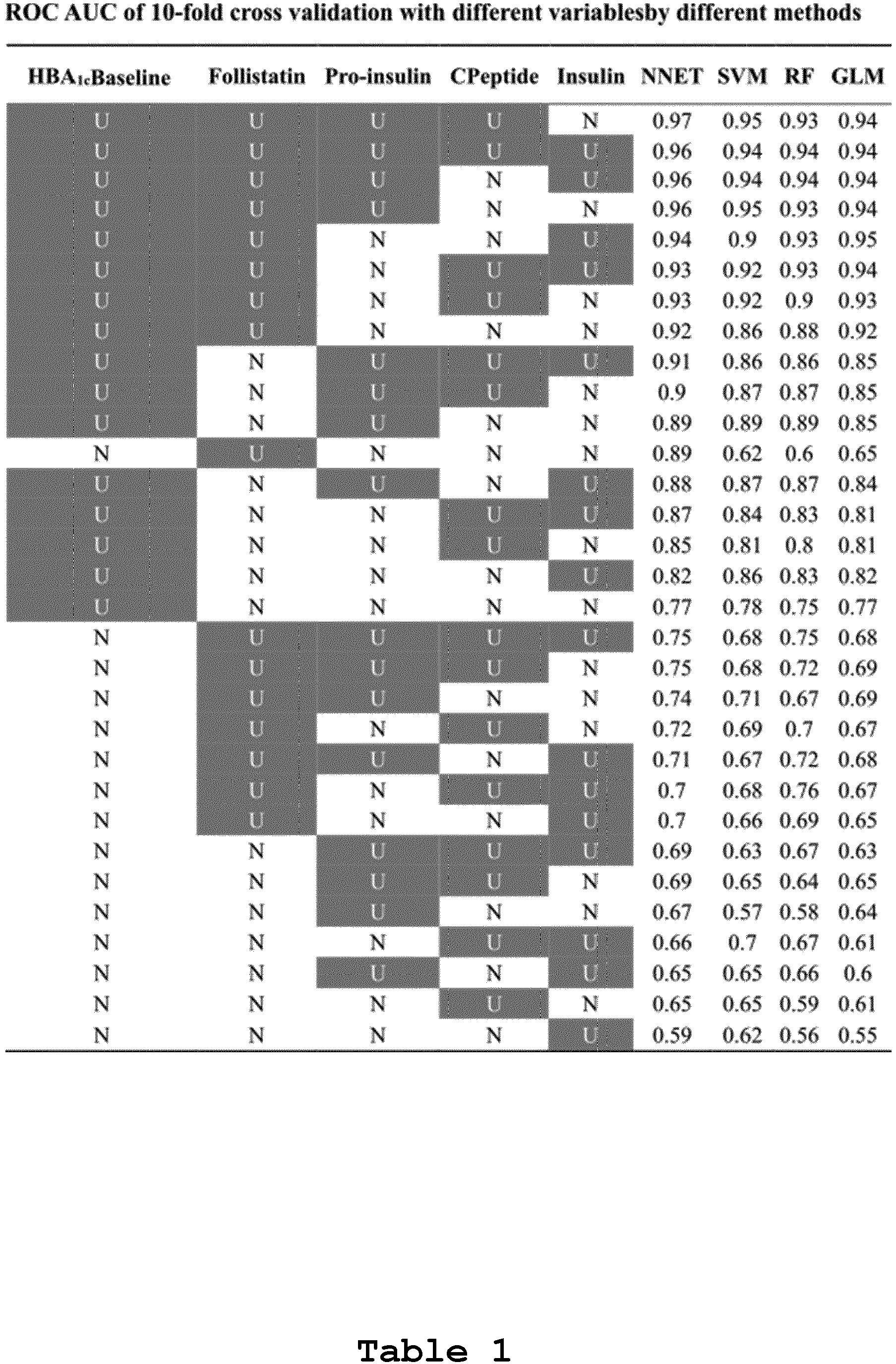

**Table 2. Performance of each cluster with 10-fold cross validation.**

| | **Sensitivity** | **Specificity** | **Accuracy** | **AUC** |
|---|---|---|---|---|
| **High Risk** | 0.7 | 0.97 | 0.835 | 0.9 |
| **Intermediate Risk** | 0.919 | 0.944 | 0.932 | 0.96 |
| **Low Risk** | 0.914 | 0.902 | 0.908 | 0.99 |
| **Overall** | 0.845 | 0.939 | 0.889 | 0.969 |

### Table 3. Comparison of AUCs between models with and without follistatin for each class

**Table 3**

| **Method** | **High Risk** | **Intermediate Risk** | **Low Risk** |
|---|---|---|---|
| **NNET** | 0.9 | 0.99 | 0.96 |
| **NNET_withoutFST** | 0.75 | 0.96 | 0.95 |
| **P value** | 4e-04 | 0.01 | 0.1 |

## Claims

1. Use of biomarker follistatin in combination with at least one further biomarker selected from baseline HbA_{1c}, proinsulin, or C-peptide in an in vitro/ex vivo method of diagnosing and/or predicting of short-term, high-risk development of type 2 diabetes in a human, wherein short term is within less than 10 years, preferably in less than 5 years, more preferably in less than 4 years.

2. Use according to claim 1, wherein the method of diagnosing and/or predicting of short-term, high-risk, development of type 2 diabetes in a human comprises k-means clustering to assess type 2 diabetes progression risk levels using at least one further biomarker selected from baseline HbA_{1c}, proinsulin, or C-peptide.

3. Use according to any one of previous claims, wherein the method of diagnosing and/or predicting of short-term, high-risk, development of type 2 diabetes in a human comprises evaluating available biomarkers by recursive feature elimination for building a risk prediction model.

4. Use according to any one of previous claims, wherein the method of diagnosing and/or predicting of short-term, high-risk, development of type 2 diabetes in a human comprises measuring blood levels from said human of follistatin and at least one further biomarker selected from baseline HbA_{1c}, proinsulin, or C-peptide, and comparing the measured blood levels with a model value based on averaged blood level values from a group of humans having a high risk of developing type 2 diabetes in less than 10 years, preferably in less than 5 year, or more preferably, in less than 4 years.

5. Use according to claim 4, wherein the method of diagnosing and/or predicting of short-term, high-risk, development of type 2 diabetes in a human comprises measuring blood levels of at least two, or at least 3further biomarkers selected from of baseline HbA_{1c}, proinsulin, or C-peptide.

6. Use according to any one of previous claim, wherein the method of diagnosing and/or predicting of short-term, high-risk, development of type 2 diabetes in a human is a method of diagnosing short-term, high-risk, development of type 2 diabetes in a human.

7. Use according to any one of the claims 1 to 6, wherein the method of diagnosing and/or predicting of short-term, high-risk, development of type 2 diabetes in a human is a method of predicting short-term, high-risk, development of type 2 diabetes in a human.

8. A method of composing a biomarker signature for the early prediction of type 2 diabetes in a human comprising measuring blood levels of follistatin in a sample obtained from said human and at least one further biomarker selected from baseline HbA_{1c}, proinsulin, or C-peptide, and comparing the measured blood levels with a model value based on averaged blood level values from a group of humans having a high risk of developing type 2 diabetes in less than 10 years, preferably in less than 5 year, or more preferably, in less than 4 years.

9. A method of composing a biomarker signature for early prediction of type 2 diabetes in a human according to claim 8, comprising measuring blood levels of at least two, or at least 3 further biomarkers selected from of baseline HbA_{1c}, proinsulin, or C-peptide.

## Patentansprüche

1. Verwendung des Biomarkers Follistatin in Kombination mit mindestens einem weiteren Biomarker, der aus Basis-HbA_{1c}, Proinsulin oder C-Peptid ausgewählt ist, in einem In-vitro-/Ex-vivo-Verfahren zum Diagnostizieren und/oder Vorhersagen einer kurzfristigen, risikoreichen Entwicklung von Typ-2-Diabetes bei einem Menschen, wobei kurzfristig innerhalb von weniger als 10 Jahren, bevorzugt in weniger als 5 Jahren, weiter bevorzugt in weniger als 4 Jahren ist.

2. Verwendung nach Anspruch 1, wobei das Verfahren zum Diagnostizieren und/oder Vorhersagen einer kurzfristigen, risikoreichen Entwicklung von Typ-2-Diabetes bei einem Menschen k-Means-Clustering zum Bewerten von Risikoniveaus einer Progredienz von Typ-2-Diabetes unter Verwendung mindestens eines weiteren Biomarkers, der aus Basis-HbA_{1c}, Proinsulin oder C-Peptid ausgewählt ist, umfasst.

3. Verwendung nach einem der vorhergehenden Ansprüche, wobei das Verfahren zum Diagnostizieren und/oder Vorhersagen einer kurzfristigen, risikoreichen Entwicklung von Typ-2-Diabetes bei einem Menschen Beurteilen verfügbarer Biomarker durch rekursive Merkmalseliminierung zum Aufbauen eines Risikovorhersagemodells umfasst.

4. Verwendung nach einem der vorhergehenden Ansprüche, wobei das Verfahren zum Diagnostizieren und/oder Vorhersagen einer kurzfristigen, risikoreichen Entwicklung von Typ-2-Diabetes bei einem Menschen Messen von Blutspiegeln von dem Menschen an Follistatin und mindestens einem weiteren Biomarker, der aus Basis-HbA_{1c}, Proinsulin oder C-Peptid ausgewählt ist, und Vergleichen der gemessenen Blutspiegel mit einem Modellwert auf Grundlage von gemittelten Blutspiegelwerten von einer Gruppe von Menschen, die ein hohes Risiko für das Entwickeln von Typ-2-Diabetes in weniger als 10 Jahren, bevorzugt in weniger als 5 Jahren oder weiter bevorzugt in weniger als 4 Jahren aufweisen, umfasst.

5. Verwendung nach Anspruch 4, wobei das Verfahren zum Diagnostizieren und/oder Vorhersagen einer kurzfristigen, risikoreichen Entwicklung von Typ-2-Diabetes bei einem Menschen Messen von Blutspiegeln an mindestens zwei oder mindestens 3 weiteren Biomarkern, die aus Basis-HbA_{1c}, Proinsulin oder C-Peptid ausgewählt sind, umfasst.

6. Verwendung nach einem der vorhergehenden Ansprüche, wobei das Verfahren zum Diagnostizieren und/oder Vorhersagen einer kurzfristigen, risikoreichen Entwicklung von Typ-2-Diabetes bei einem Menschen ein Verfahren zum Diagnostizieren einer kurzfristigen, risikoreichen Entwicklung von Typ-2-Diabetes bei einem Menschen ist.

7. Verwendung nach einem der Ansprüche 1 bis 6, wobei das Verfahren zum Diagnostizieren und/oder Vorhersagen einer kurzfristigen, risikoreichen Entwicklung von Typ-2-Diabetes bei einem Menschen ein Verfahren zum Vorhersagen einer kurzfristigen, risikoreichen Entwicklung von Typ-2-Diabetes bei einem Menschen ist.

8. Verfahren zum Erstellen einer Biomarkersignatur zur frühen Vorhersage von Typ-2-Diabetes bei einem Menschen, umfassend Messen von Blutspiegeln an Follistatin in einer von dem Menschen erlangten Probe und mindestens einem weiteren Biomarker, der aus Basis-HbA_{1c}, Proinsulin oder C-Peptid ausgewählt ist, und Vergleichen der gemessenen Blutspiegel mit einem Modellwert auf Grundlage von gemittelten Blutspiegelwerten von einer Gruppe von Menschen, die ein hohes Risiko für das Entwickeln von Typ-2-Diabetes in weniger als 10 Jahren, bevorzugt in weniger als 5 Jahren oder weiter bevorzugt in weniger als 4 Jahren aufweisen.

9. Verfahren zum Erstellen einer Biomarkersignatur zur frühen Vorhersage von Typ-2-Diabetes bei einem Menschen nach Anspruch 8, umfassend Messen von Blutspiegeln an mindestens zwei oder mindestens 3 weiteren Biomarkern, die aus Basis-HbA_{1c}, Proinsulin oder C-Peptid ausgewählt sind.

## Revendications

1. Utilisation d'un biomarqueur follistatine en combinaison avec au moins un autre biomarqueur choisi parmi l'HbA1c de ligne de base, la pro-insuline ou le peptide C dans une méthode in vitro / ex vivo de diagnostic et / ou de prédiction du développement à court terme et à haut risque du diabète de type 2 chez un être humain, le court terme étant inférieur à 10 ans, de préférence inférieur à 5 ans, et de préférence inférieur à 4 ans.

2. Utilisation selon la revendication 1, dans laquelle la méthode de diagnostic et / ou de prédiction du développement à court terme et à haut risque du diabète de type 2 chez un être humain comprend le regroupement de k-moyennes pour évaluer les niveaux de risque de progression du diabète de type 2 à l'aide d'au moins un autre biomarqueur choisi parmi l'HbA_{1c} de ligne de base, la pro-insuline ou le peptide C.

3. Utilisation selon l'une quelconque des revendications précédentes, dans laquelle la méthode de diagnostic et / ou de prédiction du développement à court terme et à haut risque du diabète de type 2 chez un être humain comprend l'évaluation de biomarqueurs disponibles par élimination de caractéristique récursive pour construire un modèle de prédiction de risque.

4. Utilisation selon l'une quelconque des revendications précédentes, dans laquelle la méthode de diagnostic et / ou de prédiction du développement à court terme et à haut risque du diabète de type 2 chez un humain comprend la mesure des niveaux sanguins dudit humain de follistatine et d'au moins un autre biomarqueur choisi parmi l'HbA_{1c} de base, la pro-insuline ou le peptide C, et la comparaison des niveaux sanguins mesurés avec une valeur modèle basée sur les valeurs moyennes des niveaux sanguins d'un groupe d'humains présentant un risque élevé de développer un diabète de type 2 dans moins de 10 ans, de préférence dans moins de 5 ans, ou plus préférablement, dans moins de 4 ans.

5. Utilisation selon la revendication 4, dans laquelle la méthode de diagnostic et / ou de prédiction du développement à court terme et à haut risque du diabète de type 2 chez un être humain comprend la mesure des niveaux sanguins d'au moins deux, ou d'au moins 3 autres biomarqueurs choisis parmi l'HbA_{1c} de ligne de base, la pro-insuline ou le peptide C.

6. Utilisation selon l'une quelconque des revendications précédentes, dans laquelle la méthode de diagnostic et / ou de prédiction du développement à court terme et à haut risque du diabète de type 2 chez un être humain est une méthode de diagnostic du développement à court terme et à haut risque du diabète de type 2 chez l'homme.

7. Utilisation selon l'une quelconque des revendications 1 à 6, dans laquelle la méthode de diagnostic et / ou de prédiction du développement à court terme et à haut risque du diabète de type 2 chez un être humain est une méthode de prédiction du développement à court terme et à haut risque du diabète de type 2 chez l'homme.

8. Procédé de composition d'une signature de biomarqueurs pour la prédiction précoce du diabète de type 2 chez un être humain, comprenant la mesure des niveaux sanguins de follistatine dans un échantillon obtenu à partir dudit être humain et au moins un autre biomarqueur choisi parmi l'HbA_{1c} de base, la pro-insuline ou le peptide C, et la comparaison des niveaux sanguins mesurés avec une valeur modèle basée sur les valeurs moyennes des niveaux sanguins d'un groupe d'humains présentant un risque élevé de développer un diabète de type 2 dans moins de 10 ans, de préférence dans moins de 5 ans, ou plus préférablement, dans moins de 4 ans.

9. Procédé de composition d'une signature de biomarqueurs pour la prédiction précoce du diabète de type 2 chez un être humain selon la revendication 8, comprenant la mesure des taux sanguins d'au moins deux, au moins 3 autres biomarqueurs choisis parmi l'HbA_{1c} de ligne de base, la pro-insuline ou le peptide C.
